# EUROPEAN PATENT SPECIFICATION

(11) **EP 1 862 137 B1**
(45) Date of publication and mention of the grant of the patent: **25.05.2011**
(21) Application number: 07010673.7
(22) Date of filing: 30.05.2007
(51) Int. Cl.: A61B 18/12, A61B 17/00, A61B 18/14

(54) **System for controlling tissue heating rate prior to cellular vaporization**
System zur Regelung der Gewebeerwärmungsgeschwindigkeit vor der Zellverdampfung
Système de contrôle de la vitesse de chauffage d'un tissu avant une vaporisation cellulaire

(30) Priority: 30.05.2006 US 442785
(43) Date of publication of application: 05.12.2007
(73) Proprietor: Covidien AG, 8212 Neuhausen am Rheinfall (CH)
(72) Inventor: Buysse, Steven P., Longmont CO 80501 (US); Weinberg, Craig, Denver CO 80211 (US)
(74) Representative: HOFFMANN EITLE

(56) References cited:
- EP-A- 1 151 725
- EP-A- 1 810 630
- US-A1- 2003 158 551
- US-A1- 2005 101 951

## Description

### BACKGROUND

### Technical Field

The present invention relates to a system for performing electrosurgical procedures. More particularly, the present invention relates to a system for controlling the heating rate of tissue prior to cellular vaporization by adjusting output of an electrosurgical generator based on sensed tissue feedback.

### Background of Related Art

Energy-based tissue treatment is well known in the art. Various types of energy (e.g., electrical, ultrasonic, microwave, cryo, heat, laser, etc.) are applied to tissue to achieve a desired result. Electrosurgery involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

Ablation is most commonly a monopolar procedure that is particularly useful in the field of cancer treatment, where one or more RF ablation needle electrodes (usually of elongated cylindrical geometry) are inserted into a living body. A typical form of such needle electrodes incorporate an insulated sheath from which an exposed (uninsulated) tip extends. When an RF energy is provided between the return electrode and the inserted ablation electrode, RF current flows from the needle electrode through the body. Typically, the current density is very high near the tip of the needle electrode, which tends to heat and destroy surrounding issue.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes. When the electrodes are sufficiently separated from one another, the electrical circuit is open and thus inadvertent contact with body tissue with either of the separated electrodes does not cause current to flow.

EP 1 810 630 was cited as prior art under Article 54 (3) EPC during examination of this patent. A system for performing and termination electrosurgical treatment in an impedance feedback algorithm is disclosed. US 2005/0101951 discloses a vessel sealing system. The preamble of claim 1 is based on this document.

It is known in the art that sensed tissue feedback may be used to control delivery of electrosurgical energy. Therefore, a need exists to develop an electrosurgical system that allows for precisely controlling output of an electrosurgical generator based on sensed tissue feedback.

### SUMMARY

There is disclosed a system and method for controlling energy output of an electrosurgical generator during initial phases of energy application. A method is not recited in the claims and thus a method does not form an embodiment of the present invention. In particular, the system generates a desired impedance trajectory including a plurality of target impedance values, based on either dynamically obtained or predefined variables. Thereafter, the system monitors tissue impedance and adjusts output of the electrosurgical generator to match tissue impedance to corresponding target impedance values.

The present invention provides an electrosurgical generator according to claim 1 and an electrosurgical system according to claim 3.

According to one aspect of the present disclosure, an electrosurgical system is disclosed. The system includes an electrosurgical generator adapted to supply electrosurgical energy at an output level to tissue and to transmit an interrogatory signal to obtain initial tissue impedance and to derive a starting impedance value. The electrosurgical generator includes a microprocessor adapted to generate a desired impedance trajectory as a function of either of the initial tissue impedance or the starting impedance value. The desired impedance trajectory includes a plurality of target impedance values. The microprocessor is further adapted to drive tissue impedance along the desired impedance trajectory by adjusting the output level to substantially match tissue impedance to a corresponding target impedance value. The system also includes an electrosurgical instrument including at least one active electrode adapted to apply electrosurgical energy to tissue.

Also disclosed in a method for performing an electrosurgical procedure is disclosed. A method is not recited in the claims and thus does not form an embodiment of the present invention. The method includes the steps of applying electrosurgical energy at an output level to tissue from an electrosurgical generator and transmitting an interrogatory signal to obtain initial tissue impedance to derive a starting impedance value. The method also includes the step of generating a desired impedance trajectory as a function of either the initial tissue impedance or the starting impedance value, wherein the desired impedance trajectory includes a plurality of target impedance values. The method further includes the step of driving tissue impedance along the desired impedance trajectory by adjusting the output level to substantially match tissue impedance to a corresponding-target impedance value.

According to a further aspect of the present disclosure, an electrosurgical generator is disclosed. The electrosurgical generator includes sensor circuitry adapted to supply energy at an output level to tissue. The electrosurgical generator is adapted to transmit an interrogatory signal to obtain initial tissue impedance to derive a starting impedance value. The electrosurgical generator also includes a microprocessor adapted to generate a desired impedance trajectory as a function of either the initial tissue impedance or the starting impedance value, wherein the desired impedance trajectory includes a plurality of target impedance values. The electrosurgical generator is adapted to drive tissue impedance along the desired impedance trajectory by adjusting the output level to substantially match tissue impedance to a corresponding target impedance value.

### BRIEF DESCRIPTION OF THE DRAWINGS

Various embodiments of the present disclosure are described herein with reference to the drawings wherein:
Fig. 1 is a schematic block diagram of an electrosurgical system according to one embodiment of the present disclosure;
Fig. 2 is a schematic block diagram of a generator according to one embodiment of the present disclosure;
Fig. 3 is a flow diagram illustrating a method which does not form part of the present invention;
Fig. 4 is an illustrative graph of impedance versus time showing the changes in impedance that occur within tissue during application of RF energy thereto; and
Fig. 5 is an illustrative graph of impedance versus time showing the changes in impedance that occur within tissue during application of RF energy thereto.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail. Those skilled in the art will understand that the method disclosed herein, which method does not form an aspect of the present invention, may be adapted to monitor use with either monopolar or bipolar electrosurgical systems.

The methods may be extended to other tissue effects and energy-based modalities, including, but not limited to, ultrasonic, laser, microwave, and cryo tissue treatments. The disclosed methods are also based on impedance measurement and monitoring but other suitable tissue and energy properties may be used to determine state of the tissue, such as temperature, current, voltage, power, energy, phase of voltage and current. The method may be carried out using a feedback system incorporated into an electrosurgical system or may be a stand-alone modular embodiment (e.g., removable modular circuit configured to be electrically coupled to various components, such as a generator, of the electrosurgical system).

A method, which does not form an aspect of the present invention, controls rate of tissue changes during preheating and/or early desiccation phases that occur prior to vaporization of intra-cellular and/or extra-cellular fluids by matching tissue impedance to target impedance based on desired rate of change of impedance over time. Hence, a method according to present disclosure may be utilized with feedback control methods that adjust energy output in response to measured tissue impedance. In particular, energy output may be adjusted before tissue phase transition to control the rate of desiccation and vaporization phases.

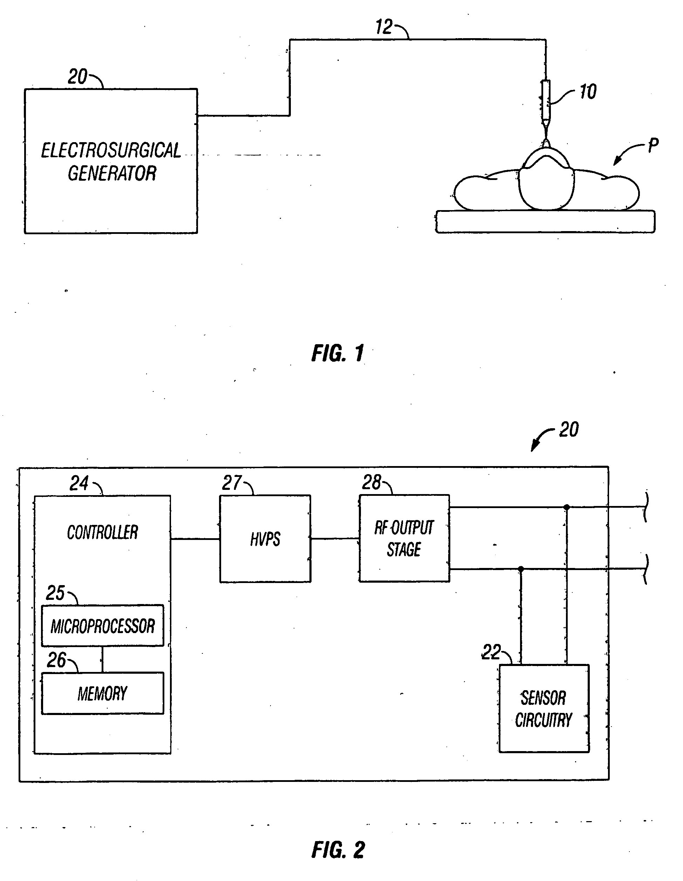

Fig. 1 is a schematic illustration of an electrosurgical system according to one embodiment of the present disclosure. The system includes an electrosurgical instrument 10 having one or more electrodes for treating tissue of a patient P. The instrument 10 may be either of monopolar type including one or more active electrodes (e.g., electrosurgical cutting probe, ablation electrode(s), etc.) or of bipolar type including one or more active and return electrodes (e.g., electrosurgical sealing forceps). Electrosurgical RF energy is supplied to the instrument 10 by a generator 20 via a supply line 12, which is connected to an active output terminal, allowing the instrument 10 to coagulate, seal, ablate and/or otherwise treat tissue.

If the instrument 10 is of monopolar type, then energy may be returned to the generator 20 through a return electrode (not explicitly shown), which may be one or more electrode pads disposed on the patient's body. The system may include a plurality of return electrodes that are arranged to minimize the chances of damaged tissue by maximizing the overall contact area with the patient P. In addition, the generator 20 and the monopolar return electrode may be configured for monitoring so called "tissue-to-patient" contact to insure that sufficient contact exists therebetween to further minimize chances of tissue damage.

If the instrument 10 is of bipolar type, the return electrode is disposed in proximity to the active electrode (e.g., on opposing jaws of bipolar forceps). The generator 20 may also include a plurality of supply and return terminals and a corresponding number of electrode leads.

The generator 20 includes input controls (e.g., buttons, activators, switches, touch screen, etc.) for controlling the generator 20. In addition, the generator 20 may include one or more display screens for providing the surgeon with variety of output information (e.g., intensity settings, treatment complete indicators, etc.). The controls allow the surgeon to adjust power of the RF energy, waveform, and other parameters to achieve the desired waveform suitable for a particular task (e.g., coagulating, tissue sealing, intensity setting, etc.). The instrument 10 may also include a plurality of input controls that may be redundant with certain input controls of the generator 20. Placing the input controls at the instrument 10 allows for easier and faster modification of RF energy parameters during the surgical procedure without requiring interaction with the generator 20.

Fig. 2 shows a schematic block diagram of the generator 20 having a controller 24, a high voltage DC power supply 27 ("HVPS") and an RF output stage 28. The HVPS 27 provides high voltage DC power to an RF output stage 28 which then converts high voltage DC power into RF energy and delivers the RF energy to the active electrode. In particular, the RF output stage 28 generates sinusoidal waveforms of high RF energy. The RF output stage 28 is configured to generate a plurality of waveforms having various duty cycles, peak voltages, crest factors, and other suitable parameters. Certain types of waveforms are suitable for specific electrosurgical modes. For instance, the RF output stage 28 generates a 100% duty cycle sinusoidal waveform in cut mode, which is best suited for ablating, fusing and dissecting tissue and a 1-25% duty cycle waveform in coagulation mode, which is best used for cauterizing tissue to stop bleeding.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed loop control scheme is a feedback control loop wherein sensor circuitry 22, which may include a plurality of sensors measuring a variety of tissue and energy properties (e.g., tissue impedance, tissue temperature, output current and/or voltage, etc.), provides feedback to the controller 24. Such sensors are within the purview of those skilled in the art. The controller 24 then signals the HVPS 27 and/or RF output stage 28, which then adjust DC and/or RF power supply, respectively. The controller 24 also receives input signals from the input controls of the generator 20 or the instrument 10. The controller 24 utilizes the input signals to adjust power outputted by the generator 20 and/or performs other control functions thereon.

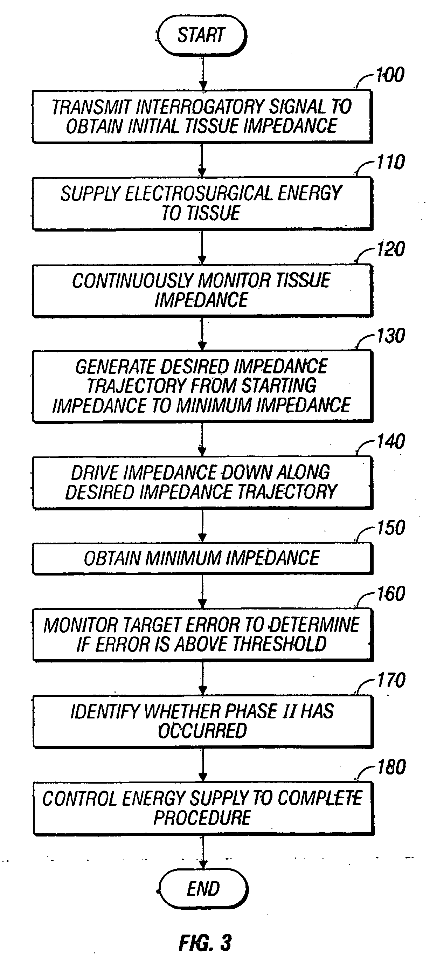

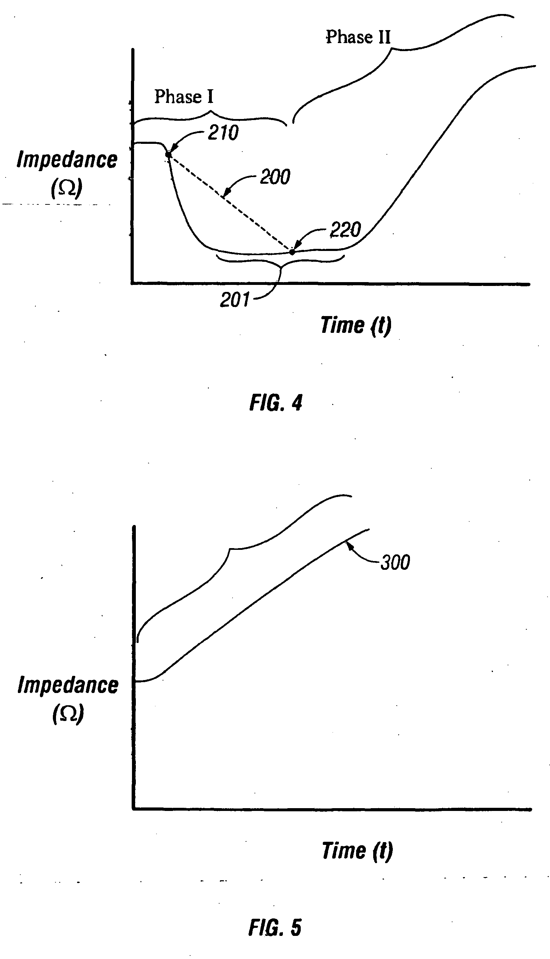

Fig. 4 shows an impedance over time graph illustrating various phases that tissue undergoes during particular application of energy thereto. The decrease in tissue impedance as energy is applied thereto occurs when tissue is being fused (e.g., vessel sealing), ablated, or desiccated. In particular, during tissue fusion, ablation, or desiccation, tissue heating results in a decreasing impedance toward a minimum value that is below the initial sensed impedance. However, tissue impedance begins to rise almost immediately when tissue is being coagulated and vaporized as shown in Fig. 5 and discussed in more detail below. The method shown in Fig. 3, which method does not form an aspect of the present invention, will now be discussed with regard to the fusion, ablation and desiccation applications.

During phase 1, which is a pre-heating or early desiccation stage, level of energy supplied to the tissue is sufficiently low and impedance of the tissue starts at an initial impedance value. As more energy is applied to the tissue, temperature therein rises and tissue impedance decreases. At a later point in-time, tissue impedance reaches a minimum impedance value 201 that correlates to tissue temperature of approximately 100° C, a boiling temperature for intra- and extra-cellular fluid boiling temperature.

Phase II is a vaporization phase or a late desiccation phase, during which tissue has achieved a phase transition from a moist, conductive to dry, non-conductive properties. In particular, as the majority of the intra- and extra-cellular fluids begin to rapidly boil during the end of phase I, impedance begins to rise above the minimum impedance value 201. As sufficient energy is continually applied to the tissue during phase II, temperature may rise beyond the boiling point coinciding with minimum impedance value 201. As impedance continues to rise, tissue undergoes a phase change from moist state to a solid state and eventually a dried-out state. As further energy is applied, tissue is completely desiccated and eventually vaporized, producing steam, tissue vapors and charring.

Previous impedance control algorithms during phase I generally applied energy uncontrollably to tissue allowing impedance to drop rapidly until reaching the minimum impedance value 201. As energy is continually delivered to tissue, the tissue can rapidly and uncontrollably transition through the minimum impedance value 201. Maintaining impedance at the minimum impedance value 201 is particularly desirable since the minimum impedance coincides with maximum conductance. Hence, it has been determined that controlling the rate at which minimum impedance value 201 provides enforced tissue effects. However, minimum impedance value 201 depends on many factors including tissue type, tissue hydration level, electrode contact area, distance between electrodes, applied energy, etc. The present invention provides a system for controlling the rate of tissue change during phase I and prior to tissue transitioning into phase II in light of these many variable tissue factors.

Fig. 3 shows a method, which method does not form an aspect of the present invention, for controlling output of the generator in response to monitored tissue impedance. In step 100, the instrument 10 is brought into a treatment site of the tissue and a low power interrogatory signal is transmitted to the tissue to obtain an initial tissue characteristic. The interrogatory signal is transmitted prior to application of electrosurgical energy. This initial tissue characteristic describes the natural tissue state and is used in subsequent calculations to determine a target slope or trajectory corresponding to a desired tissue response during phase I.

If electrosurgical energy is being used to treat the tissue, then the interrogatory signal is an electric pulse and the tissue characteristic being measured may be energy, power, impedance, current, voltage, electrical phase angle, reflected power, temperature, etc. If other energy is being used to treat tissue then the interrogatory signal and the tissue properties being sensed may be another type of interrogatory signal. For instance the interrogation signal may be achieved thermally, audibly, optically, ultrasonically, etc. and the initial tissue characteristic may then correspondingly be temperature, density, opaqueness, etc. A method is discussed using electrosurgical energy and corresponding tissue properties (e.g., impedance). Those skilled in the art will appreciate that the method may be adopted using other energy applications discussed above.

In step 110, the generator 20 supplies electrosurgical energy to the tissue through the instrument 10. In step 120, during application of energy to the tissue, impedance is continually monitored by the sensor circuitry 22. In particular, voltage and current signals are monitored and corresponding impedance values are calculated at the sensor circuitry 22 and/or at the microprocessor 25. Power and other energy properties may also be calculated based on collected voltage and current signals. The microprocessor 25 stores the collected voltage, current, and impedance within the memory 26.

In step 130, target impedance values are calculated based on the initial tissue characteristic and a desired target slope. In particular, target impedance values take the form of a desired impedance trajectory 200 when considering the position of target impedance values over time. The desired trajectory 200 is drawn to the minimum impedance value 201. More specifically, a start point 210 is defined based on the initial interrogation signal. The start point 210 is directly measured (e.g., corresponding to the initial tissue impedance) and calculated by the generator 20. The desired trajectory 200 may be predetermined value imported from a look-up table stored in memory 26 or a hard-coded input. The predetermined value and the hard-coded input may be selected based on the initial tissue impedance. Thus, the desired trajectory 200 includes a plurality of calculated target impedance values based on the desired input parameters (e.g., desired slope) from the starting point 210 to a desired end point 220 (e.g., minimum impedance value 201). The desired trajectory 200 is linear, as shown in Fig. 4.

In step 140, the generator 20 drives impedance down from starting point 210 to the minimum impedance value 201 along the desired trajectory 200 by adjusting the energy level to match measured impedance values to corresponding target impedance values. This is accomplished at specific time increments, which may be predetermined or dynamically defined. Namely, for every time increment, tissue reaction is calculated and output of the generator 20 is controlled to match measured impedance to corresponding target impedance.

As the application of energy continues adjusting its output and matching impedance along the desired trajectory 200, the generator 20 continuously monitors target error, which is the difference between target impedance value and actual impedance value. This value is used to determine the application of energy required to obtain and/or maintain a desired impedance slope. Namely, the target error represents the amount that tissue impedance deviates from a corresponding target impedance value. Hence, the energy output is adjusted based on the value of the target error. If the target error shows that measured impedance is below the target impedance, output of the generator 20 is lowered. If the target error shows that measured impedance is above the target impedance, output of the generator 20 is increased.

In step 150, during the controlled heating stage, the minimum impedance value 201 is obtained. As energy is being applied and the target and tissue impedance is being decremented, the system is continuously monitoring the tissue impedance for a minimum value. Impedance is continuously monitored by comparing a currently measured impedance value with a previously measured impedance and selecting the low of the two impedance values as the current minimum impedance.

In step 160, during the controlled heating stage, as the generator 20 drives the impedance down, target error is also being continuously monitored to determine if the error exceeds a predetermined threshold. This event helps to identify that the electrode-to-tissue interface as well as impedance is at a minimum and cannot be driven any lower. The target error may be combined with a clock timer to demark a deviation time after the target error exceeds a particular value. The minimum impedance value 201 may be considered during monitoring of the target error to determine a sustained deviation from the minimum or an instantaneous extraneous event (e.g., arcing).
During the controlled heating stage, as described in step 140, measured impedance is matched with target impedance so that tissue impedance decreases according to the desired impedance trajectory 200 until a particular tissue condition or predetermined impedance (e.g., minimum impedance value 201) is reached.

In step 170, the point of desiccation and/or vaporization in phase II is identified by an increase in impedance above the dynamically measured minimum impedance value 201 combined by a deviation from the target value. Thus, the minimum impedance value 201 and the target error are monitored and obtained in steps 150 and 160, respectively, are used to determine if tissue has progressed to the phase II. This transformation may be defined by either the threshold being above the minimum of target error and/or an absolute or relative threshold defined by the user or by other inputs, such as a look-up table based on initial interrogation information. In some embodiments, tissue and/or energy properties (e.g., energy, power, impedance, current, voltage, electrical phase angle, reflected power, temperature, etc.) are compared with reference values to identify vaporization and/or desiccation. In particular, the system is looking for the impedance to rise above a threshold and the target to deviate a dynamic or a predetermine level instantaneously and/or over a predetermined time.

Once the event coinciding with the start of desiccation and/or vaporization is identified, step 180 controls the energy to complete the treatment application (e.g., fusion, ablation, sealing, etc.). After this point, energy output is controlled to maintain minimum impedance value 201. This optimizes energy delivery by maintaining the most appropriate RF energy levels to maintain heating. Energy delivery may be controlled using existing generators and algorithms, such as Ligasure™ generators available from Valleylab, Inc. of Boulder, Colorado.

As discussed above, if the tissue impedance does not drop and in contrast begins to rise almost immediately then tissue is being coagulated and vaporized. The difference in impedance behavior is attributable to the different energy parameters associated with coagulation and vaporization. An embodiment of the method shown in Fig. 3 is particularly discussed with regard to coagulation and vaporization.

In the case of coagulation and vaporization applications, energy is applied to achieve rapid tissue phase transition (i.e., into phase II). Fig. 5 shows an impedance plot illustrating impedance changes occurring within tissue during coagulation and vaporization with impedance increasing at the onset of energy application. Hence, in energy applications where rapid tissue phase transitioning is desired, a method can also be utilized to drive impedance along a desired positive sloping trajectory 300. Such does not form an embodiment of the present invention.

The method for driving the impedance along the desired trajectory 300 is substantially similar to the method discussed above and shown in Fig. 3 with the only difference being that the desired trajectory 300 does not reach a minimum impedance and is driven along a positive slope.

Determination as to whether the impedance is to be driven either in a decreasing or increasing direction is made prior to application. Namely, the selection is made by the user based on the clinical intent (e.g., fusion, desiccation, and ablation versus coagulation and vaporization), tissue type, mode of operation, instrument type, etc.

## Claims

1. An electrosurgical generator (20) comprising sensor circuitry adapted to supply energy at an output level to tissue, the electrosurgical generator being adapted to transmit an interrogatory signal to obtain initial tissue impedance to derive a starting impedance value; and **characterized by**:
a microprocessor (25) adapted to generate a desired impedance trajectory (200) as a function of at least one of the initial tissue impedance and the starting impedance value,
wherein the desired impedance trajectory includes a plurality of target impedance values,
the electrosurgical generator being adapted to drive tissue impedance along the desired impedance trajectory by adjusting the output level to match tissue impedance to a corresponding target impedance value;
wherein the desired impedance trajectory represents a pre-desiccation phase of an electrosurgical procedure, and wherein the slope of the desired impedance trajectory includes a linear, negative slope.

2. An electrosurgical generator according to claim 1, wherein the electrosurgical generator is further adapted to monitor a target error representing the difference between a tissue impedance and the corresponding target impedance value.

3. An electrosurgical system comprising:
the electrosurgical generator according to any one of the preceding claims, which is adapted to supply electrosurgical energy to tissue and to transmit an interrogatory signal to obtain initial tissue impedance and to derive a starting impedance value; and
an electrosurgical instrument (10) including at least one active electrode adapted to apply electrosurgical energy to tissue.

## Patentansprüche

1. Elektrochirurgischer Generator (20), umfassend einen Sensorschaltkreis, der dazu angepasst ist, Energie einer Ausgangshöhe auf Gewebe abzugeben, wobei der elektrochirurgische Generator dazu angepasst ist, ein Abfragesignal zu übertragen, um eine anfängliche Gewebeimpedanz zu erhalten, um einen Anfangsimpedanzwert abzuleiten; und **gekennzeichnet durch**:
einen Mikroprozessor (25), der dazu angepasst ist, eine gewünschte Impedanztrajektorie (200) als eine Funktion der Anfangsgewebeimpedanz und/oder des Anfangsimpedanzwertes zu erzeugen, wobei die gewünschte Impedanztrajektorie mehrere Zielimpedanzwerte enthält, wobei der elektrochirurgische Generator dazu angepasst ist,
Gewebeimpedanz entlang der gewünschten Impedanztrajektorie durch Einstellen der Ausgabehöhe zu fahren, um Gewebeimpedanz mit einem entsprechenden Zielimpedanzwert in Übereinstimmung zu bringen;
wobei die gewünschte Impedanztrajektorie eine Vor-Trocknungsphase einer elektrochirurgischen Operation repräsentiert und wobei die Steigung der gewünschten Impedanztrajektorie eine lineare negative Steigung enthält.

2. Elektrochirurgischer Generator nach Anspruch 1, wobei der elektrochirurgische Generator ferner dazu angepasst ist, einen Zielfehler zu überwachen, der die Differenz zwischen einer Gewebeimpedanz und dem entsprechenden Zielimpedanzwert wiedergibt.

3. Elektrochirurgisches System, umfassend:
den elektrochirurgischen Generator nach einem der vorhergehenden Ansprüche, der dazu angepasst ist, elektrochirurgische Energie an Gewebe abzugeben und ein Abfragesignal zu übertragen, um eine Anfangsgewebeimpedanz zu erhalten und um einen Anfangsimpedanzwert abzuleiten; und
ein elektrochirurgisches Instrument (10), das zumindest eine aktive Elektrode aufweist, die dazu angepasst ist, elektrochirurgische Energie an Gewebe anzulegen.

## Revendications

1. Générateur électrochirurgical (20) comprenant un circuit de détection apte à fournir de l'énergie à un niveau de sortie au tissu, le générateur électrochirurgical étant apte à transmettre un signal d'interrogation afin d'obtenir une impédance initiale du tissu pour dériver une valeur d'impédance de départ; et **caractérisé par**:
un microprocesseur (25) apte à produire une trajectoire d'impédance souhaitée (200) en tant que fonction d'au moins une parmi l'impédance initiale du tissu et la valeur d'impédance de départ, où la trajectoire d'impédance souhaitée comporte une pluralité de valeurs d'impédance cibles, le générateur électrochirurgical étant apte à entraîner l'impédance du tissu le long de la trajectoire d'impédance souhaitée en ajustant le niveau de sortie pour faire correspondre l'impédance du tissu avec une valeur d'impédance cible correspondante;
où la trajectoire d'impédance souhaitée représente une phase de pré-dessiccation d'une procédure électrochirurgicale, et où la pente de la trajectoire d'impédance souhaitée comprend une inclinaison linéaire négative.

2. Générateur électrochirurgical selon la revendication 1, dans lequel le générateur électrochirurgical est apte en outre à surveiller une erreur cible représentant la différence entre une impédance du tissu et la valeur d'impédance cible correspondante.

3. Système électrochirurgical comprenant:
le générateur électrochirurgical selon l'une quelconque des revendications précédentes, qui est apte à fournir de l'énergie électrochirurgicale au tissu et à transmettre un signal d'interrogation pour obtenir une impédance initiale du tissu et pour dériver une valeur d'impédance de départ; et
un instrument électrochirurgical (10) comportant au moins une électrode active apte à appliquer l'énergie électrochirurgicale au tissu.
